(19) Europäisches Patentamt
European Patent Office
Office européen des brevets

(11) Publication number: 0 042 596 B1

(12) # EUROPEAN PATENT SPECIFICATION

(45) Date of publication of patent specification: 14.03.84

(21) Application number: 81104710.9

(22) Date of filing: 19.06.81

(51) Int. Cl.[3]: C 07 H 19/18, A 61 K 31/70, C 07 D 473/34, A 61 K 31/52

(54) **Novel adenine nucleoside derivatives, their preparation and pharmaceutical compositions containing them.**

(30) Priority: 23.06.80 US 162097
16.01.81 US 225567

(43) Date of publication of application:
30.12.81 Bulletin 81/52

(45) Publication of the grant of the patent:
14.03.84 Bulletin 84/11

(84) Designated Contracting States:
AT BE CH DE FR GB IT LI NL SE

(56) References cited:
FR - A - 2 351 994
US - A - 4 055 717
US - A - 4 055 718

JOURNAL OF MEDICINAL CHEMISTRY, volume 20, no. 4, April 1977 R. VINCE "Carbocyclic Arabinosyladenine, an Adenosine Deaminase Resistant Antiviral Agent" pages 612-615

The file contains technical information submitted after the application was filed and not included in this specification

(73) Proprietor: REGENTS OF THE UNIVERSITY OF MINNESOTA
University of Minnesota
Minneapolis, MN-55455 (US)

(72) Inventor: Vince, Robert
1723 Yorkshire Avenue
St. Paul Minnesota 55455 (US)

(74) Representative: Antony, Fritz, Dr. et al,
P.O. Box 601 Winkelriedstrasse 35
CH-6002 Lucerne (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Novel adenine nucleoside derivatives, their preparation and pharmaceutical compositions containing them

This invention relates to novel adenine nucleoside derivatives, to their preparation and to pharmaceutical compositions containing them. More particularly, this invention relates to novel esters of vidarabine and its carbocyclic analog cyclaradine, these esters having been found to possess useful therapeutic activity in particular useful antiviral activity.

Known in the art is the adenine nucleoside 9-(β-*D*-arabinofuranosyl)-adenine also termed vidarabine or ara-A. Vidarabine is known to exhibit antiviral activity. Also known in the art is the carbocyclic analog of vidarabine, this analog and its preparation being disclosed in United States Patent 4,138,562 and J. Med. Chem., *20*, 612 (1977). This carbocyclic analog 9 - [2α,3β - dihydroxy - 4α - (hydroxymethyl)cyclopent - 1α - yl] - adenine is also known as cyclaradine or C-ara-A. Cyclaradine has been shown to exhibit *in vitro* antiviral activity.

Also known in the art are certain simple alkanoate esters of vidarabine which are also indicated as exhibiting *in vitro* antiviral activity. Thus, French patent specification 2,351,994 discloses certain 5′-monoalkanoates of vidarabine, USP 4,055,717 certain 3′-monoalkanoates and 2′, 3′-dialkanoates of vidarabine and USP 4,055,718 certain 2′-monoalkanoates of vidarabine.

We have now found that a specified group of derivatives of the aforementioned adenine nucleosides, namely specified alkoxyalkanoates unexpectedly exhibits, inter alia, advantageous *in vivo* activity.

Accordingly, in one of its aspects the present invention provides novel adenine nucleoside derivatives of the general formula I

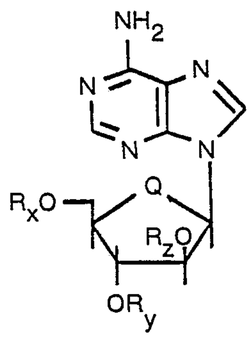

(I)

and the pharmaceutically acceptable salts thereof, wherein Q represents oxygen or methylene and $R_x$, $R_y$ and $R_z$ independently represent hydrogen or an alkoxyalkanoyl grouping $R_1$—O—$R_2$—CO—, with the proviso that at least one of $R_x$, $R_y$ and $R_z$ is the alkoxyalkanoyl grouping $R_1$—O—$R_2$—CO—, the alkyl moiety $R_1$ and the alkadiyl moiety $R_2$ each independently containing 1 to 6 carbon atoms.

The group $R_1$ may be methyl, ethyl, propyl, butyl, pentyl or hexyl, including the corresponding branched-chain isomers thereof while the group $R_2$ may be the corresponding alkadiyl analog of the aforementioned alkyl groups.

Preferably $R_x$ is $R_1$—O—$R_2$—CO— and $R_y$ and $R_z$ are hydrogen. For $R_1$ and $R_2$ preferred values are methyl and methylene respectively.

The pharmaceutically acceptable salts are those salts of the compounds of the general formula I derived from an appropriate organic or inorganic acid, such as a mineral acid. As typical salts there may be mentioned the hydrochloride and sulfate salts.

The compounds of the general formula I may exist in the form of optical isomers and these isomers as well as the racemic mixtures are included within the compounds of this invention.

Preferred compounds of the general formula I are the mtnoester derivatives, that is derivatives where $R_x$ represents $R_1$—O—$R_2$—CO— and $R_y$ and $R_z$ represent hydrogen.

Specifically preferred compounds are the 5′-methoxyacetyl ester of vidarabine and the 5′-methoxyacetyl ester of its carbocyclic analog cyclaradine alternatively named herein as 4′-methoxyacetoxymethyl cyclaradine. Other typical compounds are:

9-β-D-arabinofuranosyladenine-5′-(7-hexyloxyheptanoate);
9-β-D-arabinofuranosyladenine-5′-(4-propyloxybutyrate);
9-β-D-arabinofuranosyladenine-5′-(4-ethoxybutyrate); and
9-β-D-arabinofuranosyladenine-3′,5′-di-0-(5-ethoxyvalerate)

as well as the corresponding cyclaradine esters.

The adenine nucleoside derivatives of the general formula I may be prepared by standard esterification techniques.

Thus vidarabine or cyclaradine or a reactive derivative thereof may be reacted with the appropriate alkoxyalkanoic acid or a reactive derivative thereof, a so-obtained diester, or triester, if desired, selectively hydrolysed and then the required ester isolated in free form or in the form of a pharmaceutically acceptable salt.

Preparation of the required ester derivative of the general formula I may thus entail selectively esterifying the desired hydroxy group (or a reactive derivative thereof) of the vidarabine or cyclaradine starting material or alternatively esterifying two or more of the hydroxy groups followed by selectively hydrolysing one or, where appropriate, two of the so esterified hydroxy groups.

The precise manner of esterification in any particular instance will depend upon well-known factors such as the reactivity of the hydroxy group in question, nature of the esterifying agent, esterification conditions as well as whether a selective hydrolysis step is to be included.

As stated, in the process for preparing the compounds of the general formula I, vidarabine or cyclaradine or a reactive derivative thereof is used as starting material. The reactive derivative may be a monoester or diester derivative of vidarabine or cyclaradine falling within the general formula I, the free hydroxy group or groups constituting the reactive moiety of the vidarabine or cyclaradine derivative. The reactive derivative may also be a vidarabine or cyclaradine derivative having one or more esterified hydroxy groups capable of being replaced in a trans-esterification or like reaction by the desired ester group. As a further possibility can be mentioned the case where the reactive derivative is a halo-derivative of the parent alcohol, the desired reaction in this case being effected by reaction of that halo-derivative with an alkali metal salt of the appropriate alkoxyalkanoic acid.

Esterification of the specified started material may, as started, be effected with the appropriate alkoxyalkanoic acid or a reactive derivative thereof. In this connection, any convenient derivative may be employed, the nature of such derivative being well known to the person skilled in the art. For instance as one commonly used derivative, there may be mentioned the halide derivative of the acid in question, esterification then being effected by reacting that halide derivative with the hydroxy group of the vidarabine or cyclaradine starting material.

As will be apparent from the above description, any appropriate method for introducing the required ester function may be employed in preparing the compounds of the present invention.

By way of specific illustration, the compounds of the general formula I where $R_x$ is an alkoxyalkanoyl group and $R_y$ and $R_2$ are hydrogen may typically be prepared by adding the appropriate molar quantity of the required alkoxyalkanoyl halide to a solution of the parent vidarabine or cyclaradine in a non-reactive, non-aqueous solvent (preferably water-miscible) such as dimethylformamide, dimethylacetamide, tetrahydrofuran or pyridine. The stirred reaction mixture is then maintained at reduced temperature, say 4°C, for 18—24 hours. The reaction is then quenched by adding water or aqueous sodium bicarbonate to the reaction mixture, following which the resulting desired ester is isolated in conventional manner and purified. By using appropriate amounts of the alkoxyalkanoic acid halide one, two or three molar equivalents of the required alkoxyalkanoate moiety may be introduced into the vidarabine or cyclaradine molecule giving the monoester, diester or triester. In those instances where the immediate product is a mixture of esters, such as a mixture of a monoester and diester, separation of the required ester from the product mixture may be effected in known manner, for instance using chromatographic techniques.

Where mixed esters are required, that is a di- or tri-ester derived from more than one acid, a mono- or diester starting material may be reacted with a halide derived from a different acid to that employed in preparing the mono- or diester starting material. Further, where it is required to esterify a relatively less reactive hydroxy group, such as a secondary hydroxy group, in the presence of another hydroxy group which is relatively more reactive, such as the primary hydroxy group, that more reactive hydroxy group can be protected in known manner during the esterification and the protecting group subsequently removed.

The preparation of the compounds of the general formula I will now be illustrated by way of the following Examples:

### Example 1

9-β-D-Arabinofuranosyladenine-5′-methoxyacetate

Arabinofuranosyladenine (4.8 g, 18 mmoles) was dissolved in dry dimethylformamide (150 ml) by warming and the solution was then cooled to 0°C. Methoxyacetyl chloride (2.38 g, 22 mmoles) in dimethylformamide (20 ml) was added dropwise with stirring and the reaction mixture was stirred overnight at 4°C.

If thin-layer chromatography indicates a significant amount of remaining vidarabine, methoxyacetyl chloride (1 g) in dimethylformamide is added and the mixture is allowed to stir for an additional 18 hours at 4°C.

Ice water (20 ml) containing sodium bicarbonate (2.38 g.) was added to neutralize the solution. The solvents were removed under reduced pressure and the residue was stirred in 15% methanol in chloroform. The inorganic solid was removed by filtration and the filtrate was applied to a silica gel column and eluted with 15% methanolic chloroform. The major product fraction was evaporated and the residue was dissolved in water and refrigerated overnight. The white solid product was removed by filtration and gave 3.9 g (yield 59%) of the title compound: m.p. 74°C. Elemental Analysis: Calculated for $C_{13}H_{17}N_5O_6 \cdot \frac{1}{2}H_2O$: C, 44.82; H, 20.10; N, 5.21. Found: C, 44.66; H, 19.96; N, 5.18.

Example 2

9-$\beta$-D-Arabinofuranosyladenine-3',5'-di-methoxyacetate

The diester was prepared by the same procedure as described in example 1, using 18 mmoles of arabinofuranosyladenine and 40 mmoles of methoxyacetyl chloride and allowing the reaction mixture to stir at 4°C. overnight and then at 25°C. for an additional 24 hours.

The major fraction was obtained from a silica gel column using the same workup procedure described in example 1.

Example 3

9-$\beta$-D-Arabinofuranosyladenine-2',3',5'-tri-methoxyacetate

The triester was prepared by the same procedure as described in example 1, using 18 mmoles of arabinofuranosyladenine and 60 mmoles of methoxyacetyl chloride. The reaction mixture was stirred at 4°C overnight and then at 25°C for an additional 24 hours. The major fraction was obtained from a silica gel column using the same workup procedure described in example 1.

Example 4

9-[2$\alpha$,3$\beta$-dihydroxy-4$\alpha$(methoxyacetoxymethyl)cyclopent-1$\alpha$-yl]adenine. (Racemate).

To a solution of cyclaradine (0.106 mole) in dimethylformamide (750 ml) was added methoxyacetyl chloride (0.109 mole) in dimethylformamide (140 ml). The reaction mixture was stirred at 4°C. overnight and then water (50 ml) and sodium bicarbonate (.27 mole) was added. The volatile materials were removed *in vacuo* and the residue was applied to a silica gel column. Elution of the major fraction with methanol/chloroform (1.5/10) gave the pure title compound in 60% yield. Crystallization from a methanol/chloroform/hexane/solvent mixture followed by drying *in vacuo* at 80°C gave the product: m.p. 172—174°C. Elemental Analysis: Calculated for $C_{14}H_{19}N_5O_5$: C, 49.84; H, 5.68; N, 20.76. Found: C, 49.76; H, 5.70; N, 20.83. Crystallization from water followed by drying *in vacuo* at room temperature gave the dihydrate, m.p. 78—80°C. Elemental Analysis: Calculated for $C_{14}H_{19}N_5O_5.2H_2O$: C, 45.03; H, 6.20; N, 18.75. Found: C, 45.15; H, 6.12; N, 19.00.

Example 5

9-[2$\alpha$-hydroxy-3$\beta$-methoxyacetoxy-4$\alpha$(methoxyacetoxymethyl)cyclopent-1$\alpha$-yl)-adenine

To a solution of cyclaradine (0.106 mole) in dimethylformamide (750 ml) was added methoxyacetyl chloride (0.212 mole) in dimethylformamide (140 ml). The reaction mixture was stirred at 4°C overnight and then water (50 ml) and sodium bicarbonate (0.330 mole) was added. The volatile materials were removed *in vacuo* and the residue was applied to a silica gel column. The column was eluted with methanol/chloroform (1/10) and the first major fraction was collected and evaporated. Crystallization of the product from ethyl acetate gave the title compound as white solid, m.p. 182—184°C. Elemental Analysis: Calculated for $C_{17}H_{23}N_5O_7$: C, 49.87; H, 5.66; N, 17.11. Found: C, 50.01; H, 5.70; N, 17.26.

Example 6

9-[2$\alpha$,3$\beta$-dimethoxyacetoxy-4$\alpha$(methoxy-acetoxymethyl)cyclopent-1$\alpha$-yl]adenine

The title compound was prepared by the same procedure as described in Example 5, using, however, 0.106 mole of cyclaradine and 0.318 moles of methoxyacetyl chloride.

In the manner described in the foregoing Examples the following representative vidarabine compounds may similarly be prepared:

9-$\beta$-D-arabinofuranosyladenine-5'-(7-hexyloxyheptanoate);
9-$\beta$-D-arabinofuranosyladenine-5'-(4-propyloxybutyrate);
9-$\beta$-D-arabinofuranosyladenine-5'-(4-ethoxybutyrate); and
9-$\beta$-D-arabinofuranosyladenine-3',5'-di-O-(5-ethoxyvalerate)

as well as the corresponding cyclaradine compounds.

As previously stated, the compounds of the general formula I have been found to exhibit, *inter alia*, particularly useful *in vivo* antiviral activity. Accordingly, the compounds of the general formula I are indicated as being useful in the treatment of infections, particularly those of mammals, caused by DNA-containing viruses such as herpes viruses,.including types 1 and 2 and herpes zoster as well as in the treatment of infections caused by adenoviruses, papovaviruses (causing warts), picodnaviruses and poxviruses.

The *in vivo* antiviral activity of the compounds of the general formula I may be illustrated by way of data obtained with the following two compounds:

Compound A: Vidarabine 5'-methoxyacetate (Prepared in Example 1)

Compound B: Cyclaradine 5'-methoxyacetate (Prepared in Example 4).

The antiviral efficacy of these compounds in the treatment of genital infections in female guinea pigs was determined, the infecting virus being herpes simplex virus type 3. The *in vivo* efficacy was

measured in terms of the effect of these compounds in suppressing lesion development induced by the infecting virus. The incidence of lesions for virus-infected animals treated with the test compound was measured and compared with the results obtained for virus-infected control animals treated with placebo, or with the results obtained for untreated virus-infected animals. In the control group of animals small lesions usually appeared 3 to 4 days following infection, the virus-induced lesions reaching a maximum severity around 7 days postinfection this peak being followed by scab formation and then healing.

In each case the test animals were infected by intravaginal inoculation with the herpes simplex virus type 2 in 0.1 ml of cell culture medium.

0.1 ml of a 0.8% agarose gel containing the test compound at the stated concentration was then administered intravaginally, treatment commencing (unless otherwise stated) 3 hours postinfection and the test compound being administered 3 times daily for 7 days. The incidence of lesions was observed at regular interval and noted.

The results obtained for Compound A are given in Table 1 and for Compound B in Table 2.

TABLE 1
In vivo antiviral efficacy (Compound A)

| Test group of animals | Percentage of animals with lesions at day 7 |
|---|---|
| (i) Control group — untreated | 83 |
| (ii) Control group — placebo treated | 95 |
| (iii) Test group treated with Compound A at a concentration of: | |
| 5% | 20 |
| 10% | 30 |
| 15% | 0 |

Table 1 shows that compound A at a concentration of 15% completely suppressed lesion formation.

In a separate comparative experiment, vidarabine itself was employed in the above test procedure and compared with untreated and placebo-treated control groups of animals. The vidarabine composition was in the form of a 5% vidarabine formulation in 0.8% agarose gel. At day 4 postinfection, 50% of the animals treated with vidarabine had lesions and at day 7 postinfection, 80% had lesions. For the control groups at day 7, the untreated control group showed 85% of the animals with lesions and the placebo-treated control group showed 90% with lesions.

TABLE 2
In vivo antiviral efficacy (Compound B)

| Test group of animals | Percentage of animals with lesions at day 5 |
|---|---|
| (i) Control group — untreated | 60 |
| (ii) Control group — placebo treated | 90 |
| (III) Test group treated with Compound B at a concentration of: | |
| 5% | 33 |
| 10% | 33 |

For the results given in Table 2, drug administration was commenced 6 hours postinfection and the test compound administered three times daily for 5 days.

As can be seen from Table 2, 60—90% of the herpes simplex type 2-infected control animals developed virus-induced lesions compared with 33% of the Compound B-treated animals.

In a separate comparative experiment, cyclaradine was employed in the above test procedure. At concentrations of 3% and 10%, the group treated with cyclaradine showed 100% of the test animals had lesions at day 7 postinfection as had an untreated control group and a placebo-treated control group.

From the above *in vivo* results, it will be seen that the esters of the general formula I were surprisingly more active than the parent alcohols vidarabine and cyclaradine.

In addition to the above-indicated particularly useful *in vivo* antiviral activity, the compounds of the general formula I where Q is $-CH_2-$ have the further advantage of being resistant to degradation by the enzyme adenosine deaminase (a normal constituent of human serum).

Further, the compounds of the general formula I are indicated as being more water-soluble than the parent vidarabine and cyclaradine compounds and this higher solubility has the added advantage of facilitating formulation of the active compounds in creams or other aqueous formulations. Thus, at 37°C in water, cyclaradine has a solubility of 1 mg/ml while cyclaradine 5'-methoxy-acetate has a solubility of 75 mg/ml.

Regarding toxicity, the compounds of the invention are indicated as being no more toxic than the parent compounds vidarabine (literature $LD_{50}$ 4,700 mg/kg i.p.) and cyclaradine. Thus in comparative testing in random-bred Swiss mice treated i.p., cyclaradine was not lethal at a dose of 900 mg/kg while for cyclaradine 5'-methoxyacetate no signs of toxicity at all were noted at the highest dose tested of 750 mg/kg.

The alkoxyalkanoate esters of the formula I can be formulated in standard fashion with conventional pharmaceutical excipients. Accordingly, in another of its aspects, the present invention provides pharmaceutical compositions comprising as active ingredient a compound of the general formula I together with a suitable pharmaceutical carrier.

Illustrative of such compositions are topical formulations where an amount of from about 1 to 20% by weight of the ester may be used. For application to the skin the concentration is desirably in the range of from about 5 to 20% by weight in the case of vidarabine esters of the general formula I, preferably 10 to 15%. In the case of cyclaradine esters of the general formula I, an amount of 1—5% by weight of the ester may be used with a preferred value being about 2.5%. In any particular instance the appropriate amount of active ingredient may be ascertained. For the formulation bases, standard dermatological cream and ointment bases can be employed in the usual manner. For treatment of susceptible viral infections of the eye or genital areas, standard ophthalmic and vaginal bases, respectively, such as creams or solutions can be employed.

In a typical regimen, topically acceptable formulations are applied four times daily to the affected site for a period of five to fourteen days until the infection clears. These compositions can be applied to the infected site in the usual manner. Semi-solid dosage forms can be spread manually or with an applicator, and liquid forms can be applied by dropper or spray.

The compounds of the general formula I may also be administered parenterally, in parenterally acceptable vehicles, in the treatment of herpes virus, encephalitis, shingles, disseminated varicella, cytomegalovirus infection, and the like.

Typical pharmaceutical compositions containing a compound of the general formula I as active ingredient are illustrated by way of the following one-gram topical formulation examples containing 15% of active ingredient, the preparation of the formulation employing standard techniques.

Example 7

Topical Cream

| | |
|---|---|
| Vibarabine-5'-methoxy-acetate | 150 mg |
| Propylene glycol | 100 mg |
| 4-chloro-m-cresol | 1 mg |
| Sodium phosphate, monobasic monohydrate | 2.7 mg |
| Phosphoric acid | 0.02 mg |
| white petrolatum | 150 mg |
| Polyethylene glycol monocetyl ether | 18 mg |
| Cetostearyl alcohol | 72 mg |
| Mineral oil | 60 mg |
| q.s. water; purified U.S.P. | |

Example 8
Topical Ointment

| | | |
|---|---|---|
| Vidarabine-5′-methoxy-acetate | 150 | mg |
| Mineral oil | 50 | mg |
| q.s. white petrolatum | | |

Example 9
Ophthalmic Ointment

| | | |
|---|---|---|
| Vidarabine-5′-methoxy-acetate | 150 | mg |
| Methyl paraben | 0.5 | mg |
| Propyl paraben | 0.1 | mg |
| q.s. white petrolatum | | |

Corresponding 2.5% and 5% cyclaradine formulations may be prepared by replacing the vidarabine 5′-methoxyacetate active ingredient in the above examples by 25 mg and 50 mg respectively of cyclaradine 5′-methoxyacetate.

**Claims for the Contracting States: BE CH DE FR GB IT LI NL SE**

1. An adenine nucleoside derivative of the general formula I

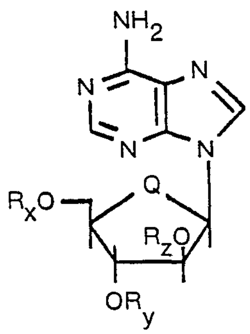

(I)

and the pharmaceutically acceptable salts thereof, wherein Q represents oxygen or methylene and $R_x$, $R_y$ and $R_z$ independently represent hydrogen or an alkoxyalkanoyl grouping $R_1$—O—$R_2$—CO—, with the proviso that at least one of $R_x$, $R_y$ and $R_z$ is the alkoxyalkanoyl grouping $R_1$—O—$R_2$—CO—, the alkyl moiety $R_1$ and the alkadiyl moiety $R_2$ each independently containing 1 to 6 carbon atoms.

2. A compound as claimed in claim 1, wherein $R_x$ represents a said alkoxyalkanoyl grouping and $R_y$ and $R_z$ are hydrogen.

3. A compound as claimed in claim 1, wherein $R_x$ and $R_y$ each represent a said alkoxyalkanoyl grouping and $R_z$ represents hydrogen.

4. A compound as claimed in claim 1, wherein $R_x$, $R_y$ and $R_z$ each represent a said alkoxyalkanoyl grouping.

5. A compound as claimed in any one of the preceding claims, wherein the alkoxyalkanoyl grouping is a methoxyacetyl grouping.

6. A compound as claimed in any one of the preceding claims, wherein Q is oxygen.

7. A compound as claimed in any one of claims 1 to 5, wherein Q is methylene.

8. The 5′-monomethoxyacetyl ester of vidarabine.

9. The 5′-monomethoxyacetyl ester of cyclaradine.

10. A process for the preparation of an alkoxy alkanoate ester of the general formula I set forth in claim 1, which process comprises reacting vidarabine or cyclaradine or a reactive derivative thereof with the appropriate alkoxyalkanoic acid or a reactive derivative thereof, if desired selectively hydrolysing the so-obtained compound, and then isolating the required ester in free form or in the form of a pharmaceutically acceptable salt.

11. A process as claimed in claim 10, wherein vidarabine or cyclaradine is reacted with the appropriate molar quantity of an alkoxyalkanoic acid halide.

12. A pharmaceutical composition comprising as active ingredient a compound as claimed in any one of claims 1 to 9 together with a suitable pharmaceutical carrier.

**Claims for the Contracting State: AT**

1. A process for the preparation of an adenine nucleoside derivative of the general formula I

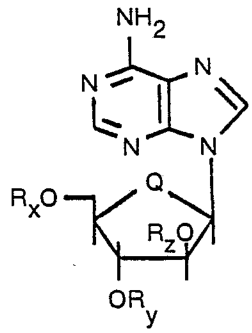

(I)

and the pharmaceutically acceptable salts thereof, wherein Q represents oxygen or methylene and $R_x$, $R_y$ and $R_z$ independently represent hydrogen or an alkoxyalkanoyl grouping $R_1$—O—$R_2$—CO—, with the proviso that at least one of $R_x$, $R_y$ and $R_z$ is the alkoxyalkanoyl grouping $R_1$—O—$R_2$—CO—, the alkyl moiety $R_1$ and the alkadiyl moiety $R_2$ each independently containing 1 to 6 carbon atoms, which comprises esterifying vidarabine or cyclaradine, or a reactive derivative thereof, with an alkoxyalkaxoic acid of the general formula $R_1$—O—$R_2$—COOH, or a reactive derivative thereof, wherein $R_1$ and $R_2$ are as defined for formula I, if desired, selectively hydrolysing a so-obtained diester or triester, and then isolating the so-obtained ester of the general formula I in free form or in the form of a pharmaceutically acceptable salt.

2. A process as claimed in claim 1, wherein the free vidarabine or cyclaradine starting material is esterified with an alkoxyalkanoyl halide, preferably chloride, of the general formula $R_1$—O—$R_2$—COHal where Hal represents halogen.

3. A process as claimed in claim 1 or claim 2, wherein the alkoxyalkanoyl grouping $R_1$—O—$R_2$—CO— is a methoxyacetyl group.

4. A process as claimed in any one of the preceding claims, which comprises esterifying the vidarabine or cyclaradine starting material with 1 molar equivalent of the said alkoxyalkanoic acid, or reactive derivative thereof, and the so-obtained 5′-monoester of the general formula is isolated as such or in the form of a phrmaceutically acceptable salt.

5. A process as claimed in any one of the preceding claims, wherein vidarabine or cyclaradine is reacted with a methoxyacetyl halide and the so-obtained 5′-methoxyacetate ester of vidarabine or cyclaradine is isolated.

6. A process for the preparation of a pharmaceutical composition which comprises admixing a compound of the general formula I set forth in claim 1 with a suitable pharmaceutically acceptable carrier.

7. A process as claimed in claim 6, wherein the compound of the general formula I set forth in claim 1 has been prepared by a process as claimed in any one of claims 1 to 5.

**Patentansprüche für die Vertragsstaaten: BE CH DE FR GB IT LI NL SE**

1. Adeninnucleosid-Derivat der allgemeinen Formel I

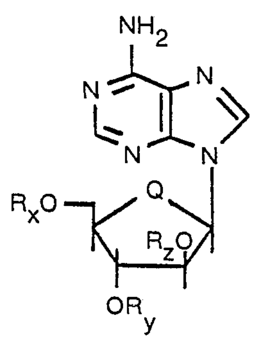

(I)

in der

Q Sauerstoff oder Methylen und

$R_x$, $R_y$ und $R_z$ unabhängig voneinander Wasserstoff oder eine Alkoxyalkanoyl-Gruppierung $R_1$—O—$R_2$—CO— bezeichnen, mit der Maßgabe, daß wenigstens einer der Substituenten $R_x$, $R_y$ und $R_z$ die Alkoxyalkanoyl-Gruppierung $R_1$—O—$R_2$—CO— ist, wobei die Alkyl-Struktureinheit $R_1$ und die

Alkadiyl-Struktureinheit $R_2$ jeweils unabhängig voneinander 1 bis 6 Kohlenstoff-Atome enthalten, und dessen pharmazeutisch unbedenkliche Salze.

2. Verbindung nach Anspruch 1, dadurch gekennzeichnet, daß $R_x$ eine solche Alkoxyalkanoyl-Gruppierung bezeichnet und $R_y$ und $R_z$ Wasserstoff sind.

3. Verbindung nach Anspruch 1, dadurch gekennzeichnet, daß $R_x$ und $R_y$ jeweils eine solche Alkoxyalkanoyl-Gruppierung bezeichnen und $R_z$ Wasserstoff bezeichnet.

4. Verbindung nach Anspruch 1, dadurch gekennzeichnet, daß $R_x$, $R_y$ und $R_z$ jeweils eine solche Alkoxyalkanoyl-Gruppierung bezeichnen.

5. Verbindung nach irgendeinem der vorhergehenden Ansprüche, dadurch gekennzeichnet, daß die Alkoxyalkanoyl-Gruppierung eine Methoxyacetyl-Gruppierung ist.

6. Verbindung nach irgendeinem der vorhergehenden Ansprüche, dadurch gekennzeichnet, daß Q Sauerstoff ist.

7. Verbindung nach einem der Ansprüche 1 bis 5, dadurch gekennzeichnet, daß Q Methylen ist.

8. Vidarabin-5'-monomethoxyacetylester.

9. Cyclaradin-5'-monomethoxyacetylester.

10. Verfahren zur Herstellung eines Alkoxyalkanoat-Esters der in Anspruch 1 angegebenen allgemeinen Formel I, dadurch gekennzeichnet, daß Vidarabin oder Cyclaradin oder ein reaktionsfähiges Derivat derselben mit der entsprechenden Alkoxyalkan-Säure oder einem reaktionsfähigen Derivat derselben umgesetzt werden, eine so erhaltene Verbindung gewünschtenfalls selektiv hydrolysiert wird und dann der benötigte Ester in freier Form oder in Form eines pharmazeutisch unbedenklichen Salzes isoliert wird.

11. Verfahren nach Anspruch 10, dadurch gekennzeichnet, daß Vidarabin oder Cyclaradin mit der geeigneten Stoffmenge (molaren Menge) eines Alkoxyalkansäurehalogenids umgesetzt werden.

12. Pharmazeutische Zusammensetzung, enthaltend als Wirkstoff eine Verbindung nach irgendeinem der Ansprüche 1 bis 9 zusammen mit einem pharmazeutisch unbedenklichen Träger.

**Patentansprüche für den Vertragsstaat: AT**

1. Verfahren zur Herstellung eines Adeninnucleosid-Derivats der allgemeinen Formel I

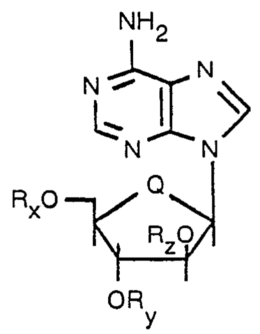

(I)

in der

Q Sauerstoff oder Methylen und

$R_x$, $R_y$ und $R_z$ unabhängig voneinander Wasserstoff oder eine Alkoxyalkanoyl-Gruppierung $R_1$—O—$R_2$—CO— bezeichnen, mit der Maßgabe, daß wenigstens einer der Substituenten $R_x$, $R_y$ und $R_z$ die Alkoxyalkanoyl-Gruppierung $R_1$—O—$R_2$—CO— ist, wobei die Alkyl-Struktureinheit $R_1$ und die Alkadiyl-Struktureinheit $R_2$ jeweils unabhängig voneinander 1 bis 6 Kohlenstoff-Atome enthalten, und dessen pharmazeutisch unbedenklicher Salze,

dadurch gekennzeichnet, daß Vidarabin oder Cyclaradin oder ein reaktionsfähiges Derivat derselben mit einer Alkoxyalkan-Säure der allgemeinen Formel $R_1$—O—$R_2$—COOH oder einem reaktionsfähigen Derivat derselben, worin $R_1$ und $R_2$ die für die Formel I definierte Bedeutung haben, verestert werden, ein so erhaltener Diester oder Triester gewünschtenfalls selektiv hydrolysiert wird und dann der so erhaltene Ester der allgemeinen Formel I in freier Form oder in Form eines pharmazeutisch unbedenklichen Salzes isoliert wird.

2. Verfahren nach Anspruch 1, dadurch gekennzeichnet, daß das freie Vidarabin- oder Cyclaradin-Ausgangsmaterial mit einem Alkoxyalkanoylhalogenid der allgemeinen Formel $R_1$—O—$R_2$—COHal, in der Hal für Halogen steht, vorzugsweise mit dem Chlorid, verestert wird.

3. Verfahren nach Anspruch 1 oder 2, dadurch gekennzeichnet, daß die Alkoxyalkanoyl-Gruppierung $R_1$—O—$R_2$—CO— eine Methoxyacetyl-Gruppe ist.

4. Verfahren nach irgendeinem der vorhergehenden Ansprüche, dadurch gekennzeichnet, daß das Vidarabin- oder Cyclaradin-Ausgangsmaterial mit 1 Stoffmengen-Äquivalent (molarem Äquivalent) der Alkoxyalkan-Säure oder dem reaktionsfähigen Derivat derselben verestert wird und der so erhaltene 5'-Monoester der allgemeinen Formel als solcher oder in der Form eines pharmazeutisch unbedenklichen Salzes isoliert wird.

5. Verfahren nach irgendeinem der vorhergehenden Ansprüche, dadurch gekennzeichnet, daß Vidarabin oder Cyclaradin mit einem Methoxyacetylhalogenid umgesetzt werden und die so erhaltenen 5'-Methoxyacetat-Ester des Vidarabins oder Cyclaradins isoliert werden.

6. Verfahren zur Herstellung einer pharmazeutischen Zusammensetzung, dadurch gekennzeichnet, daß eine Verbindung der in Anspruch 1, angegebenen allgemeinen Formel I mit einem geeigneten, pharmazeutisch unbedenklichen Träger vermischt wird.

7. Verfahren nach Anspruch 6, dadurch gekennzeichnet, daß die Verbindung der in Anspruch 1 angegebenen allgemeinen Formel I hergestellt ist mittels eines Verfahrens nach einem der Ansprüche 1 bis 5.

**Revendications pour les Etats contractants: BE CH DE FR GB IT LI NL SE**

1. Dérivé d'adénine nucléoside de formule générale I

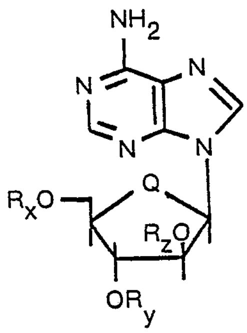

(I)

et ses sels acceptables en pharmacie, où Q représente de l'oxygène ou du méthylène et $R_x$, $R_y$ et $R_z$ représentent indépendamment de l'hydrogène ou un groupement alcoxyalcanoyle $R_1$—O—$R_2$—CO—, à condition qu'au moins l'un de $R_x$, $R_y$ et $R_z$ soit le groupement alcoxyalcanoyle $R_1$—O—$R_2$—CO—, le fragment alcoyle $R_1$ et le fragment alcadiyle $R_2$ contenant chacun indépendamment 1 à 6 atomes de carbone.

2. Composé selon la revendication 1, où $R_x$ représente un groupement alcoxyalcanoyle et $R_y$ et $R_z$ sont de l'hydrogène.

3. Composé selon la revendication 1, où $R_x$ et $R_y$ représentent chacun un groupement alcoxyalcanoyle et $R_z$ représente de l'hydrogène.

4. Composé selon la revendication 1, où $R_x$, $R_y$ et $R_z$ représentent chacun un groupement alcoxyalcanoyle.

5. Composé selon l'une quelconque des revendications précédentes, où le groupement alcoxyalcanoyle est un groupement méthoxyacétyle.

6. Composé selon l'une quelconque des revendications précédentes, où Q est de l'oxygène.

7. Composé selon l'une quelconque des revendications 1 à 5, où Q est du méthylène.

8. 5'-monométhoxyacétyl ester de vidarabine.

9. 5'-monométhoxyacétyl ester de cyclaradine.

10. Procédé de préparation d'un ester d'alcoxyalcanoate de formule générale I selon la revendication 1, lequel procédé consiste à faire réagir de la vidarabine ou de la cyclaradine ou son dérivé réactif avec l'acide alcoxyalcanoïque approprié ou son dérivé réactif, à hydrolyser sélectivement, si on le souhaite, le composé ainsi obtenu puis à isoler l'ester requis sous forme libre ou sous la forme d'un sel acceptable en pharmacie.

11. Procédé selon la revendication 10, où l'on fait réagir la vidarabine ou la cyclaradine avec la quantité molaire appropriée d'un halogénure d'acide alcoxyalcanoïque.

12. Composition pharmaceutique comprenant comme ingrédient actif un composé selon l'une quelconque des revendications 1 à 9 avec un véhicule pharmaceutique approprié.

**Revendications pour l'Etat contractant AT**

1. Procédé de préparation d'un dérivé d'adénine nucléoside de formule générale I

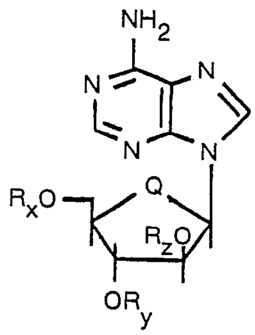

(I)

et ses sels acceptables en pharmacie, où Q représente de l'oxygène ou du méthylène et $R_x$, $R_y$ et $R_z$ représentent indépendamment de l'hydrogène ou un groupement alcoxyalcanoyle $R_1$—O—$R_2$—CO—, à condition qu'au moins l'un de $R_x$, $R_y$ et $R_z$ soit le groupement alcoxyalcanoyle $R_1$—O—$R_2$—CO—, le fragment alcoyle $R_1$ et le fragment alcadiyle $R_2$ contenant chacun indépendamment de 1 à 6 atomes de carbone,
qui consiste à extérifier de la vidarabine ou de la cyclaradine ou son dérivé réactif, avec un acide alcoxyalcaxoïque de formule générale $R_1$—O—$R_2$—COOH, ou son dérivé réactif, où $R_1$ et $R_2$ sont tels que définis pour la formule I, si on le souhaite, à hydrolyser sélectivement un diester ou triester ainsi obtenu puis à isoler l'ester ainsi obtenu de formule générale I sous sa forme libre ou sous la forme de son sel acceptable en pharmacie.

2. Procédé selon la revendication 1, où la matière première libre de vidarabine ou cyclaradine est estérifiée avec un halogénure d'alcoxyalcanoyle, de préférence un chlorure, de formule générale $R_1$—O—$R_2$—COHal où Hal représente un halogène.

3. Procédé selon la revendication 1 ou la revendication 2, où le groupement alcoxyalcanoyle $R_1$—O—$R_2$—CO— est un groupe méthoxyacétyle.

4. Procédé selon l'une quelconque des revendications précédentes qui consiste à estérifier la matière première de vidarabine ou cyclaradine avec un équivalent molaire dudit acide alcoxyalcanoique ou son dérivé réactif, et le 5'-monoester de formule générale ainsi obtenu est isolé tel quel ou sous la forme d'un sel acceptable en pharmacie.

5. Procédé selon l'une quelconque des revendications précédentes où l'on fait réagir la vidarabine ou la cyclaradine avec un halogénure de méthoxyacétyle et l'ester de 5'-méthoxyacétate de vidarabine ou cyclarabine ainsi obtenu est isolé.

6. Procédé pour la préparation d'une composition pharmaceutique qui consiste à mélanger un composé de formule générale I indiqué à la revendication 1 avec un véhicule approprié acceptable en pharmacie.

7. Procédé selon la revendication 6, où le composé de formule générale I indiqué à la revendication 1 a été préparé par un procédé selon l'une quelconque des revendications 1 à 5.